# EUROPÄISCHE PATENTSCHRIFT

(11) **EP 1 259 312 B1**
(45) Veröffentlichungstag und Bekanntmachung des Hinweises auf die Patenterteilung: **27.05.2009**
(21) Anmeldenummer: 01905812.2
(22) Anmeldetag: 21.02.2001
(51) Int. Cl.: B01J 2/02, B01J 2/18, A61K 8/00

(54) **FESTSTOFFGRANULATE MIT MONODISPERSER KORNGRÖSSENVERTEILUNG**
GRANULAR SOLID WITH MONODISPERSE PARTICLE SIZE DISTRIBUTION
GRANULATS SOLIDES AYANT UNE REPARTITION GRANULOMETRIQUE MONODISPERSEE

(30) Priorität: 02.03.2000 DE 10009996
(43) Veröffentlichungstag der Anmeldung: 27.11.2002
(73) Patentinhaber: Cognis IP Management GmbH, 40589 Düsseldorf (DE)
(72) Erfinder: SCHMID, Karl, Heinz, 40822 Mettmann (DE); WOLLMANN, Gerhard, 40723 Hilden (DE); NEUSS, Michael, 50997 Köln (DE); GUTSCHE, Bernhard, 40724 Hilden (DE); ROEGEL, Gilbert, F-77840 Crouy sur Ourcq (FR)
(86) Internationale Anmeldenummer: PCT/EP2001/001927
(87) Internationale Veröffentlichungsnummer: WO 2001/064326

(56) Entgegenhaltungen:
- WO-A-99/33555
- DE-A- 3 035 331
- DE-B- 2 725 924
- US-A- 5 674 504

## Beschreibung

### Gebiet der Erfindung

Die Erfindung befindet sich auf dem Gebiet der Kosmetik und betrifft leichtlösliche Feststoffe mit einer monodispersen Korngrößenverteilung, ein Verfahren zu ihrer Herstellung sowie ihre Verwendung zur Herstellung von oberflächenaktiven Zubereitungen, speziell Mitteln zur Haar- und Körperpflege.

### Stand der Technik

Die Konfektionierung von bei Raumtemperatur festen kosmetischen Einsatzstoffen, wie beispielsweise Emulgatoren, Wachskörpem oder Konsistenzgebern, erfolgt dabei in der Regel durch Sprühkristallisation. Üblicherweise sind im Kopf der Sprühtürme mehrere Einstoff-Druckdüsen, sogenannte Vollkegeldüsen installiert, über die die Einsatzstoffe in den Sprühturm zerstäubt werden. Dabei reißt der mit hoher Turbulenz aus der Düse austretende Strahl schon nach kurzer Entfernung von der Düsenmündung auf und bildet Tropfen, wobei das Aufreißen durch den Drall des Strahls noch zusätzlich verstärkt wird. Das auf diese Art und Weise hergestellte Kristallisationsprodukt weist ein breites Korngrößenspektrum auf. Aufgrund des hierbei anfallenden Feinstaubanteils und der Gefahr einer Staubexplosion, unterliegen derartige Sprühanlagen den erweiterten Pflichten eines Störfallbetriebes, was nicht unerhebliche Anforderungen an die Arbeitssicherung beinhaltet und zu einer ökonomischen Belastung des Herstellverfahrens führt. Ein weiteres Problem besteht in der Tendenz der Sprühkristallisate zusammenzubacken, was den Aufwand beim Einarbeiten in die Endformulierungen erheblich erhöht.

US 5,674,504 beschreibt kosmetische Gele, die sphärische Partikel eines nicht-hydrophilen lipidartigen Feststoffes enthalten.

DE 2723924 beschreibt ein Verfahren zur Herstellung kugelförmiger Teilchen aus niedrigschmelzenden organischen Trägersubstanzen mit darin gelösten oder dispergierten Wirkstoffen.

WO 00/33555 beschreibt eine Apparatur sowie eine Verfahren zur Herstellung kugelförmiger Teilchen aus einer Schmelze über eine Gießerplatte, wobei die Schmelze über eine Gießerplatte in Vibration versetzt wird. In diesem Verfahren wird das Kühlmittel, nicht entgegengeleitet, sondern bereits beim Vertropfen der Schmelze zugeführt.

Bei den Herstellern von Kosmetikrohstoffen besteht daher ein lebhaftes Interesse an Feststoffen mit einem eingeengten Korngrößenspektrum, insbesondere mit einem vernachlässigbarem Feinstkorn- bzw. Staubanteil (< 0,5 mm) von unter 1 Gew.-%. Gleichzeitig besteht der Wunsch nach solchen Produkten, die sich in öliger wie auch wässriger Phase rascher lösen und somit leichter einarbeitbar sind.

Die Aufgabe der vorliegenden Erfindung hat daher darin bestanden, neue Feststoffgranulate aus mindestens einem kosmetischen Einsatzstoff ausgewählt aus der Gruppe nichtionischer Tenside, die gebildet wird von Alkyl- und/oder Alkenyloligoglykosiden, Fettsäure-N-alkylpoly-hydroxyalkylamiden, Fettalkoholpolyglycolethern, Mischethern, alkoxylierten Fettsäurealkylestern und Polyolpoly-12-hydroxystearaten, zur Verfügung zu stellen, welche frei von den geschilderten Nachteilen sind, d.h. eine monodisperse Korngrößenverteilung, praktisch keinen Staubanteil und eine verbesserte Auflösegeschwindigkeit sowohl in wässrigem wie auch öligem Medium aufweisen.

### Beschreibung der Erfindung

Gegenstand der Erfindung sind Feststoffgranulate mit einer monodispersen Korngrößenverteilung, die dadurch erhältlich sind, dass man eine bei Raumtemperatur feste, überwiegend wasserfreie Zubereitung aus mindestens einem kosmetischen Einsatzstoff verflüssigt, einen Strom der Schmelze mit Hilfe einer Gießerplatte durch Vibration vertropft und den Tropfen ein Kühlmittel entgegenleitet, welches diese zum Erstarren bringt.

Überraschenderweise wurde gefunden, dass das gesteckte Ziel durch eine gegenüber der konventionellen Sprühkristallisation geänderte Tropfenerzeugung zu erreichen ist. Hierbei werden die Einsatzgemische als Schmelze über einen Lochteller oder eine Düsenplatte in den Vertropfungsturm eingebracht. Durch eine angeregte Membran wird der Flüssigkeit eine Frequenz aufgeprägt, der Flüssigkeitsfaden immer wieder unterbrochen und es bilden sich aufgrund der Grenzflächenspannung Kügelchen, die anschließend in den eigentlichen Sprühturm gelangen und dann während des freien Falls beispielsweise durch einen entfeuchteten Kaltluftgegenstrom zur Kristallisation gebracht werden. In Abhängigkeit von Lochdurchmesser und Schwingfrequenz der Membran kann ein definiertes Komspektrum ohne Staubanteil eingestellt werden. Eine derartige Anlage unterliegt damit nicht mehr den erweiterten Pflichten eines Störfallbetriebes, was zu einer drastischen Verminderung des technischen Aufwandes und der damit verbundenen Kosten führt. Bei der Herstellung der Granulate besteht zudem in der Regel ein breiter Erstarrungsbereich. Die Produkte besitzen häufig eine Phase als unterkühlte Flüssigkeiten, so dass die Nachkristallisation mit einer Wärmetönung verbunden ist. Hierdurch kann eine Blockbildung durch Verkleben der Teilchen verhindert werden. Ein wesentlicher Vorteil besteht schließlich darin, dass die so erhaltenen "Perlen" gegenüber konventionellen Anbietungsformen, beispielsweise Pellets oder Schuppen, eine signifikant verbesserte Löslichkeit sowohl in öligen als wässrigen Medien zeigen. Dies begründet im Übrigen den Anspruch, dass es sich um neue Stoffe handelt, die ausgewählt sind aus der Gruppe, die gebildet wird von Alkyl- und/oder Alkenyloligoglykosiden, Fettsäure-N-alkylpoly-hydroxyalkylamiden, Fettalkoholpolyglycolethern, Mischethern, alkoxylierten Fettsäurealkylestern und Polyolpoly-12-hydroxystearaten.

Ein weiterer Gegenstand der Erfindung betrifft ein Verfahren zur Herstellung von Feststoffgranulaten mit einer monodispersen Korngrößenverteilung, bei dem man eine bei Raumtemperatur feste, überwiegend wasserfreie Zubereitung aus mindestens einem kosmetischen Einsatzstoff verflüssigt, einen Strom der Schmelze mit Hilfe einer Gießerplatte durch Vibration vertropft und den Tropfen ein Kühlmittel entgegenleitet, welches diese zum Erstarren bringt.

### Kosmetische Einsatzstoffe

Die Auswahl der kosmetischen Einsatzstoffe ist an sich unkritisch, solange diese in wasserfreier Form bei Temperaturen unter 25 °C, vorzugsweise bei 18 bis 22 °C als Feststoffe vorliegen. Vorzugsweise kommen als Einsatzstoffe Emulgatoren, Wachskörper und Konsistenzgeber sowie deren Gemische in Frage. Diese werden in der Regel als wasserfreie Zubereitungen eingesetzt, aufgeschmolzen und dann vertropft. Es ist jedoch auch möglich solche Zubereitungen einzusetzen, welche überwiegend wasserfrei sind, d.h. bei denen der Wasseranteil unter 50, vorzugsweise unter 25 und insbesondere unter 5 Gew.-% liegt. Dies ist beispielsweise dann der Fall, wenn anionische oder nichtionische Emulgatoren zum Einsatz gelangen, welche herstellungsbedingt oftmals als wäßrige Tensidpasten vorliegen.

### Emulgatoren

Als Emulgatoren kommen beispielsweise nichtionogene Tenside aus mindestens einer der folgenden Gruppen in Frage:
➢ Alkyl- und/oder Alkenyloligoglykoside, Fettsäure-N-alkylpolyhydroxyalkylamide;
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Mischether und ethoxylierte Fettsäurealkylester;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß DE 1165574 **PS** und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

Alkyl- und Alkenyloligoglykoside stellen bekannte nichtionische Tenside dar, die der Formel **(I)** folgen,

**R¹O-[G]ₚ** **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften EP-A1 0301298 und WO 90/03977 verwiesen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside.** Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohiol, Erucylalkohol, Brassidylalkohol sowie deren technische Gemische, die wie oben beschrieben erhalten werden können. Bevorzugt sind Alkyloligoglucoside auf Basis von linearen oder verzweigten Fettalkoholen mit 8 8 bis 18 bzw. 16 bis 18 bzw. Kohlenstoffatomen, insbesondere technischer Kokosfettalkohol bzw. Cetearylalkohol oder Isostearylalkohol.

Fettsäure-N-alkylpolyhydroxyalkylamide stellen ebenfalls geeignete nichtionische Emulgatoren dar, die vorzugsweise der Formel **(II)** folgen, 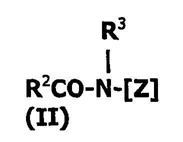 in der R²CO für einen aliphatischen Acylrest mit 6 bis 22 Kohlenstoffatomen, R³ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften US 1,985,424**,** US 2,016,962 und US 2,703,798 sowie die Internationale Patentanmeldung WO 92/06984 verwiesen. Eine Übersicht zu diesem Thema von H.Kelkenberg findet sich in Tens.Surf. Deterg. 25, 8 (1988**).** Vorzugsweise leiten sich die Fettsäure-N-alkylpolyhy-droxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel **(III)** wiedergegeben werden:

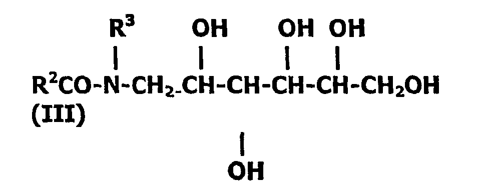

Vorzugsweise werden als Fettsäure-N-alkylpolyhydroxyalkylamide Glucamide der Formel **(III)** eingesetzt, in der R³ für eine Alkylgruppe steht und R²CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Lino-lensäure, Arachinsäure, Gadoleinsäure, Behensäure oder Erucasäure bzw. derer technischer Mischungen steht. Besonders bevorzugt sind Fettsäure-N-alkylglucamide der Formel **(III),** die durch reduktive Aminierung von Glucose mit Methylamin und anschließende Acylierung mit Palmitin-, Stearin- und/oder Isostearinsäure bzw. einem entsprechenden Derivat erhalten werden. Weiterhin können sich die Polyhydroxyalkylamide auch von Maltose und Palatinose ableiten.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Vorzugsweise handelt es sich Anlagerungsprodukte von 5 bis 50 und insbesondere 10 bis 20 Mol Ethylenoxid an Fettalkohole mit 12 bis 22 und vorzugsweise 16 bis 18 Kohlenstoffatomen.

Typische Beispiele für geeignete ethoxylierte Partialglyceride sind Anlagerungsprodukte von 1 bis 30, vorzugsweise 5 bis 10 Mol Ethylenoxid an Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind die Polyolpoly-12-hydroxystearate. Hierbei handelt es sich um bekannte Stoffe, die beispielsweise in der internationale Patentanmeldung WO 95/34528 (Henkel) beschrieben werden. Die Polyolkomponente der Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
(a) Glycerin und Polyglycerin;
(b) Alkylenglycole, wie beispielsweise Ethylenglycol, Diethylenglycol, Propylenglycol;
(c) Methyolverbindungen, wie insbesondere Trimethylolethan, Trimethylolpropan, Trimethylolbutan, Pentaerythrit und Dipentaerythrit;
(d) Alkyloligoglucoside mit 1 bis 22, vorzugsweise 1 bis 8 und insbesondere 1 bis 4 Kohlenstoffen im Alkylrest, wie beispielsweise Methyl- und Butylglucosid;
(e) Zuckeralkohole mit 5 bis 12 Kohlenstoffatomen, wie beispielsweise Sorbit oder Mannit,
(f) Zucker mit 5 bis 12 Kohlenstoffatomen, wie beispielsweise Glucose oder Saccharose;
(g) Aminozucker wie beispielsweise Glucamin.

Unter den erfindungsgemäß einzusetzenden Emulgatoren kommt Umsetzungsprodukten auf Basis von Polyglycerin wegen ihrer ausgezeichneten anwendungstechnischen Eigenschaften eine besondere Bedeutung zu. Als besonders vorteilhaft hat sich die Verwendung von ausgewählten Polyglycerinen erwiesen, die die folgende Homologenverteilung aufweisen (in Klammern angegeben sind die bevorzugten Bereiche):

| | |
|---|---|
| Glycerin : | 5 bis 35 (15 bis 30) Gew.-% |
| Diglycerine : | 15 bis 40 (20 bis 32) Gew.-% |
| Triglycerine : | 10 bis 35 (15 bis 25) Gew.-% |
| Tetraglycerine : | 5 bis 20 (8 bis 15) Gew.-% |
| Pentaglycerine : | 2 bis 10 (3 bis 8) Gew.-% |
| Oligoglycerine : | ad 100 Gew.-% |

Weitere geeignete Polyglycerinester sind Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Als nichtionische Emulgatoren kommen insbesondere auch Mischether bzw. Hydroxymischether in Frage, bei denen es sich um Umsetzungsprodukte der bereits genannten Fettalkoholpolyglycolether mit C₁-C₈ Alkylhalogeniden oder C₈-C₁₂-Epoxiden handelt. Gleichfalls geeignet sind alkoxylierte Fettsäureniedrigalkylester, die man erhält, indem man beispielsweise in die Esterbindung von C₁₂-C₁₈-Fettsäuremethylestern 1 bis 10 Mol Ethylenoxid insertiert. Dies gelingt u.a. durch Umsetzung der Ester mit Ethylenoxid in Gegenwart von calcinierten Hydrotalciten als Katalysatoren.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispiels-weise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Wachskörper

Typische Beispiele für geeignete Wachskörper, die im Sinne der Erfindung vertropft werden können, sind beispielsweise natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Ester von Fettsäuren mit Fettalkoholen, Polyalkylenwachse und Polyethylenglycolwachse in Frage.

Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Periglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber

Als wichtigste Gruppe der Konsistenzgeber, die vertropft werden können, sind die Fettalkohole zu nennen. Hierunter sind primäre aliphatische Alkohole zu verstehen, die vorzugsweise der Formel **(IV)** folgen,

**R⁴OH** **(IV)**

in der R⁴ für einen aliphatischen, linearen oder verzweigten Kohlenwasserstoffrest mit 6 bis 22 Kohlenstoffatomen und 0 und/oder 1, 2 oder 3 Doppelbindungen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische lineare und/oder verzweigte Fettalkohole mit 16 bis 18 Kohlenstoffatomen, wie beispielsweise Cetearylalkohol oder Isostearylalkohol.

Weiterhin kommen als Konsistenzgeber Partialglyceride in Frage, also Monoglyceride, Diglyceride und deren technische Gemische kommen ebenfalls als Zuschlagstoffe in Frage und können herstellungsbedingt noch geringe Mengen Triglyceride enthalten. Die Partialglyceride folgen vorzugsweise der Formel **(V),** 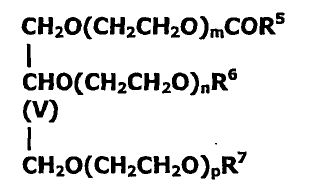 in der R⁵CO für einen linearen oder verzweigten, gesättigten und/oder ungesättigten Acylrest mit 6 bis 22, vorzugsweise 12 bis 18 Kohlenstoffatomen, R⁶ und R⁷ unabhängig voneinander für R⁵CO oder OH und die Summe (m+n+p) für 0 oder Zahlen von 1 bis 100, vorzugsweise 5 bis 25 steht, mit der Maßgabe, daß mindestens einer der beiden Reste R⁶ und R⁷ OH bedeutet. Typische Beispiele sind Mono- und/oder Diglyceride auf Basis von Capronsäure, Caprylsäure, 2-Ethylhexansäure, Caprinsäure, Laurinsäure, I-sotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise wer-den technische Cetearylglyceride, Palmitinsäureglyceride, Stearinsäureglyceride und/oder Isostearinsäureglyceride eingesetzt, welche einen Monoglyceridanteil im Bereich von 50 bis 95, vorzugsweise 60 bis 90 Gew.-% aufweisen.

### Spezielle Mischungen

In einer bevorzugten Ausführungsform der vorliegenden Erfindung werden Zuckertenside vom Typ der Alkyloligoglucoside zusammen mit Konsistenzgeber aus der Gruppe der Fettalkoholen eingesetzt, wobei es sich empfiehlt, solche Vertreter einzusetzen, die über identische Alkylreste verfügen, da diese nicht nur anwendungstechnisch Vorteile besitzen, sondern auch ökonomisch interessant sind, da sie bereits während der Synthese der Glucoside als Zwischenprodukte anfallen. Ganz besonders bevorzugt werden solche Gemische vertropft, die Alkyloligoglucoside der Formel **(I)** und Fettalkohole der Formel **(IV),** in der R¹ und R⁴ für lineare oder verzweigte Alkylreste mit 16 bis 18 Kohlenstoffatomen, speziell Cetearyl- oder Isostearylreste im Gewichtsverhältnis 10 : 90 bis 90 : 10 und speziell 40 : 60 bis 60 : 40 enthalten. Die Einstellung des Glucosid/Fettalkoholverhältnisses kann beispielsweise durch die Wahl des Fettalkoholüberschusses in der Acetalisierung eingestellt werden. Nachträglich kann Fettalkohol beispielsweise durch Extraktion im nahekritischen Bereich oder Molekulardestillation entfernt werden. Derartige Produkte - wenn auch in nicht-granularer Form - sind beispielsweise unter den Marken Emulgade® PL 68/50 oder Montanov® 68 im Handel.

In einer weiteren bevorzugten Ausführungsform werden solche Gemische vertropft, die Al- kyloligoglucoside der Formel **(I),** in der R¹ für lineare oder verzweigte Alkylreste mit 8 bis 18 Kohlenstofatomen, und Polyglycerinpoly-12-hydroxystearate im Gewichtsverhältnis 40 : 60 bis 60 : 40 und insbesondere 50 : 50 enthalten. Derartige Produkte - wenn auch in nicht-granularer Form - sind unter der Marke Eumulgin® VL 75 im Handel.

### Vertropfung

Die im Sinne des erfindungsgemäßen Verfahrens durchgeführte Vertropfung mit Hilfe einer vibrierenden Gießerplatte bzw. Gießplatte mit angeregter Membran ist bereits für die Verarbeitung von Harzen sowie niedrigviskosen Polyestern bekannt. Entsprechende Bauteile werden beispielsweise von der Firma Rieter-Automatik unter Bezeichnung "Droppo Line" für den Einsatz in der Textiltechnik vertrieben. Im Sinne des erfindungsgemäßen Verfahrens sind solche Gießerplatten bevorzugt, welche aus einer beheizbaren Ober- und Unterplatte bestehen. Die Unterplatte ist in der Regel als Lochscheibe geformt, durch deren Öffnungen oder Kanäle bzw. Kapillardüsen oder Vertropfungsröhrchen die Tropfen dann in den Sprühturm gelangen. In einer besonderen Ausgestaltung des Verfahrens sind die Düsen so ausgeformt, dass sie neben dem Eduktstrom auch die Einspeisung eines zusätzlichen Gasstroms gestatten ("Steuerluft"), mit dem die Tröpfchen weiter vernebelt werden können. Dieser Gasstrom kann beispielsweise auf 100 bis 120 °C vorgeheizt sein, so dass mit ihrer Hilfe auch noch im Edukt enthaltene Wasserspuren verdampft werden. Die Leistung solcher Lochscheiben, die üblicherweise 10 bis 750 Bohrungen aufweisen, kann vorzugsweise im Bereich von 0,3 bis 3 kg/h/Bohrung liegen, der Durchmesser der Bohrungen liegt zwischen 0,25 und 1,4 mm. Dabei werden Granulate erhalten, die einen Durchmesser vom 1,6 bis 2,7fachen des Durchmessers der Bohrungen aufweisen

Die Schwingungsfrequenz der Lochscheiben ("Tropfteller") liegt typischerweise im Bereich von 100 bis 10.000 und vorzugsweise 200 bis 800 Hz. Sie kann über eine angeregte Membran, ein schwingenden Stößel, eine vibrierende Platte oder Sonoanregung erzeugt werden.

Ein weiterer Vorteil gegenüber den herkömmlichen Verfahren besteht ferner darin, daß mit nur geringem Überdruck (typisch : 100 bis 4000 mbar) gearbeitet werden kann. Die Temperatur, mit der die Einsatzstoffe in den Vertropfungsturm gefördert werden, wird durch den Erstarrungsbereich begrenzt und liegt in der Regel im Bereich von 40 bis 100 °C, wobei darauf zu achten ist, dass die Edukte nicht überhitzt werden, da dies zu einer Zersetzung und Anbackungen führen kann. Als besonders effektiv haben sich Feedtemperaturen erwiesen, die 3 bis 10 °C über dem Erstarrungspunkt der Einsatzstoffe liegen. Üblicherweise besitzen die Edukte eine Viskosität kleiner 500 mPas (Brookfield, Spindel 1, 20 °C, 10 Upm). Die Tropfen fallen im Turm turbulenzarm senkrecht herunter. Die Wahrscheinlichkeit, dass sich mehrere Tropfen treffen und zu einem sogenannten "Himbeergranulat" zusammenbacken, ist gering. Die Kühlung kann zwar grundsätzlich auch mit kalter Flüssigkeit erfolgen, aus praktischen Gründen ist jedoch eine Kühlung im Vertropfungsturm mit kalter Luft im Gegenstrom empfehlenswert, wie dies im Stand der Technik hinreichend beschrieben wird. Die Granulate sind annähernd kugelförmig und weisen danach in Abhängigkeit der Öffnungen in der Lochplatte Und der Frequenz durchschnittliche Durchmesser im Bereich von 1 bis 2,5 mm auf. Falls gewünscht kann der Sprühturm mit einem Rüttelsiebboden ausgestattet sein, um die Korngrößenverteilung noch homogener zu gestalten.

Der Staubanteil, d.h. Partikel mit Teilchengrößen kleiner 0,3 mm ist dabei praktisch Null, daher sind keine Apparate zur Staubabtrennung aus der Ablauft erforderlich. Ohne Staub ist eine Kreisgasfahrweise möglich, was zu einer Reduzierung des Entfeuchtungsaufwandes für zugeleitete Frischluft führt. Anstelle von Luft kann wegen der Kreisgasfahrweise natürlich auch Inertgas eingesetzt werden.

Für Produkte mit Restwärme und mit Nachkristallisationswärme kann dem Vertropfungsturm eine Nachkühlstrecke nachgeschaltet sein.Als Nachkühler eignen sich beispielsweise gasdurchströmte Fließ-, Fest- oder Rutschbetten, flüssigkeitsgekühlte Wendelförderer, pneumatische Förderer oder Nachkühlbänder. Bei Produkten, die vor dem Erstarren zunächst eine unterkühlte Flüssigkeit bilden, kann die Kristallisation angeregt werden durch Zugabe von inerten Keimbildnern zur Schmelze oder in den Gasstrom, Aufzwingen einer Sekundärströmung in der Düse (Drallströmung), Durchfallen der Tropfen durch ein Sonarfeld, z.B. ein Tiefton- oder ein Ultraschallfeld oder die bewußte Erzeugung von Satellitentropfen durch Veränderung der Frequenz.

### Gewerbliche Anwendbarkeit

Ein weiterer Gegenstand der Erfindung betrifft die Verwendung der Feststoffgranulate zur Herstellung von oberflächenaktiven, vor allem kosmetischen Zubereitungen, insbesondere für den Bereich der Haut- und Haarpflegemittel. Sie können jedoch auch für den Bereich der Mund- und Zahnhygiene sowie für Wasch-, Spül-, Reinigungs- und Avivagemittel eingesetzt werden. Die Einsatzmenge der Granulate liegt im Bereich von 1 bis 70, vorzugsweise 5 bis 50 und insbesondere 15 bis 35 Gew.-% - bezogen auf die Mittel.

Diese Mittel können ferner als weitere Hilfs- und Zusatzstoffe milde Tenside, Ölkörper, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten. In einer besonderen Ausgestaltungsform der Erfindung ist es auch möglich, daß die hier genannten und im folgenden näher erläuterten Zusatzstoffe zusammen mit den kosmetischen Einsatzstoffen vertropft und dann als Mischung "Compound" eingesetzt werden. Dies trifft insbesondere für UV-Lichtschutzfilter, Antioxidantien und biogene Wirkstoffe vor, wobei auch hier wieder das Kriterium gilt, dass die Zusatzstoffe nach Möglichkeit bei Raumtemperatur fest sein sollen.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfo-fettsäuren, Alkylsulfate, Fettalkoholethersulfate, Glycerinethersulfate, Fettsäureethersulfate, Hydroxymischethersulfate, Monoglycerid-(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, bzw. Glucoronsäurederivate, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 **oder** J.Fatbe (ed.), "Katalysatoren, Tenside und Mineralöladditive", Thieme Verlag, Stuttgart, 1978, S. 123-217 verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, O-leylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. DE 19756377 A1**),** insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether, Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Verdickungsmittel

Als Verdickungsmittel kommen Hydroxyfettalkohole mit 12 bis 22 und vorzugsweise 16 bis 18 Kohlenstoffatomen und daneben Fettsäuren oder Hydroxyfettsäuren in Betracht. Geeignete Verdicker sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolldon/Vinylacrylat-Copolymere, Vinylacetat/Butylmaleat/ Isobornylacrylat-Copolymere, Methylvinylether/Maleinsäureanhydrid-Co- polymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxyproylmethacrylat-Copolymere, Polyvinyl- pyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Cellulose-ether und Silicone in Frage. Weitere geeignete Polymere und Verdickungsmittel sind in Cosmetics & Toiletries 108, 95 [1993**]** aufgeführt.

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976**).**

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der EP 0693471 B1 beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)-benzoe-säureamylester;
➢ Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenylzimtsäure-2-ethylhexyiester (Octocrylene);
➢ Ester der Salicylsäure, vorzugsweise Salicylsäure-2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester;
➢ Triazinderivate, wie z.B. 2,4,6-Trianitino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996**)** sowie Parfümerie und Kosmetik 3 (1999), Seite 11ff zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄ Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure sowie deren Fragmente, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutyl-carbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerin-monocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremono-ethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbelöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achseinässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesqui-chlorhydrat und deren Komplexverbindungen z. B. mit Propylenglycol-1,2. Alumnium-hydroxyallantoinat, Aluminiumchloridtartrat, Aluminium-Zirkonium-Trichlorohydrat, Alumi-nium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993**)** entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

### Parfümöle

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbelöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

### Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

### Beispiele

**Beispiel 1.** In einer Droppo-Line-Anlage der Fa. Rieter Automatik GmbH mit 0,3 mm-Düsen wurde eine 50:50-Mischung aus Cetearyloligoglucosid und Cetearylalkohol (Emulgade® PL 68/50, Cognis Deutschland GmbH, Schmelzpunkt 64 °C) mit einer Geschwindigkeit von 0,7 kg/h/Loch vertropft. Ausgehend von einer Schmelztemperatur von 80 °C stellte sich eine Produkttemperatur von 21°C ein. Das Abkühlen erfolgte mit 3 °C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,6 mm aufwiesen. Ein mit 780 kg/m² zusammengepreßtes Produkt ließ sich nach 2 h Lagerzeit problemlos wieder in Einzelperlen zerteilen. Das Produkt zeigte keine Temperaturerhöhung durch Restwärme. Die Auflösegeschwindigkeit in Hexyllaurat der so erhaltenen Perlen wurde im Vergleich zu kommerzieller Ware in geprillter Form wie folgt getestet: Jeweils 900 g Hexyllaurat wurden auf 60 °C erwärmt; in diesem Öl wurden unter Rühren in Mengen von 100 g jeweils die erfindungsgemäßen Perlen und zum Vergleich die kommerziell verfügbaren Schuppen gelöst. Die bestimmten Auflösegeschwindigkeiten betrugen für die erfindungsgemäßen Perlen 8 min und für die konventionelle geschuppte Ware 21 min.

**Beispiel 2.** In einer Droppo-Line-Anlage der Fa. Rieter Automatik GmbH mit 0,5 mm-Düsen wurde eine 50:50-Mischung aus Cetearyloligoglucosid und Cetearylalkohol (Emulgade® PL 68/50) mit einer Geschwindigkeit von 2,5 kg/h/Loch vertropft. Ausgehend von einer Schmelztemperatur von 80 °C stellte sich eine Produkttemperatur von 55 °C ein. Das Abkühlen erfolgte mit 10 °C kalter Luft auf einer Fallhöhe von 11 m. Es wurde ein staubfreies rieselfähiges Granulat erhalten; dieses bestand aus gleichmäßigen Perlen, die zu > 70% eine Größe von 0,95 mm aufwiesen. Ein mit 780 kg/m² zusammengepreßtes Produkt ließ sich nach 2 h Lagerzeit problemlos wieder in Einzelperlen zerteilen. Das Produkt zeigte keine Temperaturerhöhung durch Restwärme. Die bestimmten Auflösegeschwindigkeiten in Hexyllaurat betrugen für die erfindungsgemäßen Perlen 9 min und für die konventionelle geschuppte Ware 21 min.

**Beispiel 3.** In einer Droppo-Line-Anlage der Fa. Rieter Automatik GmbH mit 0,5 mm-Düsen wurde eine 50:50-Mischung aus Kokosalkyloligoglucosid und Polyglycerinpoly-12-hydroxy-stearat (Eumulgin® VL 75, Cognis Deutschland GmbH) mit einer Geschwindigkeit von 1,1 kg/h/Loch vertropft. Ausgehend von einer Schmelztemperatur von 95 °C stellte sich eine Produkttemperatur von 65 °C ein. Das Abkühlen erfolgte mit 3 °C kalter Luft auf einer Fallhöhe von 11 m. Es wurde ein staubfreies rieselfähiges Granulat erhalten, das aus gleichmäßigen Perlen bestand, welche zu > 70 % eine Größe von 0,95 mm aufwiesen.

**Beispiel 4.** In einer Droppo-Line-Anlage der Fa. Rieter Automatik GmbH mit 0,5 mm-Düsen wurde ein Cetearylalkohol (Lanette® O, Cognis Deutschland GmbH, Schmelzpunkt 51 °C) mit einer Geschwindigkeit von 2,5 kg/h/Loch vertropft. Ausgehend von einer Schmelztemperatur von 65 °C stellte sich eine Produkttemperatur von 39 °C ein. Das Abkühlen erfolgte mit 10 °C kalter Luft auf einer Fallhöhe von 11 m. Es wurde ein staubfreies rieselfähiges Granulat erhalten; dieses bestand aus gleichmäßigen Perlen, die zu > 70 % eine Größe von 1 mm aufwiesen. Ein mit 780 kg/m² zusammengepreßtes Produkt ließ sich nach 2 h Lagerzeit problemlos wieder in Einzelperlen zerteilen. Das Produkt zeigte keine Temperaturerhöhung durch Restwärme. die bestimmten Auflösegeschwindigkeiten in Hexyl-laurat betrugen für die erfindungsgemäßen Perlen 6 min und für die konventionelle geschuppte Ware 12 min.

**Beispiel 5.** In einer Droppo-Line-Anlage der Fa. Rieter Automatik GmbH mit 0,3 mm-Düsen wurde ein Glycerinmonostearat (Cutina® GMS-V, Cognis Deutschland GmbH, Schmelzpunkt 59 °C) mit einer Geschwindigkeit von 2,0 kg/h/Loch vertropft. Ausgehend von einer Schmelztemperatur von 7 °C stellte sich eine Produkttemperatur von 38 °C ein. Das Abkühlen erfolgte mit 3 °C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,65 mm aufwiesen. Die bestimmten Auflösegeschwindigkeiten betrugen für die erfindungsgemäßen Perlen 8 min und für konventionelle geschuppte Ware 19 min.

**Beispiel 6.** In einer Droppo-Line-Anlage der Fa. Rieter Automatik GmbH mit 0,3 mm-Düsen wurde eine Mischung (Schmelztemperatur 62 °C) aus (a) 100 Teilen einer 50:50-Mischung aus Cetearyloligoglucosid und Cetearylalkohol (Emulgade® PL 68/50), (b) 5 Teilen Natrium-Cetyl/stearylsulfat (Lanette® E, Cognis Deutschland GmbH) und (c) 40 Teilen Glycerinmonostearat (Cutina® GMS-V) mit einer Geschwindigkeit von 1,5 kg/h/Loch vertropft. Ausgehend von einer Schmelztemperatur von 75 °C stellte sich eine Produkttemperatur von 43 °C ein. Das Abkühlen erfolgte mit 10 °C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,65 mm aufwiesen. Die bestimmten Auflösegeschwindigkeiten bertrugen für die erfindungsgemäßen Perlen 21 min und für die konventionelle geschuppte Ware 41 min.

**Beispiel 7.** In einer Droppo-Line-Anlage der Fa. Rieter Automatik GmbH mit 0,5 mm-Düsen wurde eine Mischung (Schmelztemperatur 62°C) aus (a) 100 Teilen einer 50:50-Mischung aus Cetearyloligoglucosid und Cetearylalkohol (Emulgade® PL 68/50) und 10 Teilen Weizenproteinhydrolysat-Pulver (Gluadin® WP, Cognis Deutschland GmbH) mit einer Geschwindigkeit von 1,5 kg/h/Loch vertropft. Ausgehend von einer Schmelztemperatur von 75 °C stellte sich eine Produkttemperatur von 46 °C ein. Das Abkühlen erfolgte mit 10 °C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,95 mm aufwiesen. Die bestimmten Auflösegeschwindigkeiten betrugen für die erfindungsgemäßen Perlen 10 min und für die konventionelle geschuppte Ware 21 min.

**Beispiel 8.** In einer Droppo-Uine-Anlage der Fa. Rieter Automatik GmbH mit 0,3 mm-Düsen wurde eine Mischung (Schmelztemperatur 52 °C) aus (a) 80 Teilen Cutina® CBS (Mischung konsistenzgebender Wachse, Cognis Deutschland GmbH), (b) 15 Teilen Ceteareth-12 (Eumulgin® B 1, Cognis Deutschland GmbH) und (c) 15 Teilen Ceteareth-20 (Eumulgin® B 2, Cognis Deutschland GmbH) mit einer Geschwindigkeit von 1,5 kg/h/Loch vertropft. Ausgehend von einer Schmelztemperatur von 65°C stellte sich eine Produkttemperatur von 38°C ein. Das Abkühlen erfolgte mit 10°C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,6 mm aufwiesen. Die bestimmten Auflösegeschwindigkeiten betrugen für die erfindungsgemäßen Perlen 16 min und für die konventionelle geschuppte Ware 29 min.

**Beispiel 9.** In einer Droppo-Line-Anlage der Fa. Rieter Automatik GmbH mit 0,3 mm-Düsen wurde eine Mischung (Schmelztemperatur 55 °C) aus 80 Teilen Stearinsäuremono/ diglycerid (Cutina® MD, Cognis Deutschland GmbH), (b) 8 Teilen Ceteareth-12 (Eumulgin B1) und (c) Teilen Ceteareth-20 (Eumulgin® B 2) mit einer Geschwindigkeit von 1,5 kg/h/Loch vertropft. Ausgehend von einer Schmelztemperatur von 70 °C stellte sich eine Produkttemperatur von 45 °C ein. Das Abkühlen erfolgte mit 10 °C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,6 mm aufwiesen. Die bestimmten Auflösegeschwindigkeiten betrugen für die erfindungsgemäßen Perlen 14 min und für die konventionelle geschuppte Ware 26 min.

**Beispiel 10.** In einer Droppo-Line-Anlage der Fa. Rieter Automatik GmbH mit 0,5 mm-Düsen wurde eine Mischung (Schmelztemperatur 56 °C) aus 30 Teilen Cetearylalkohol (Lanette® O), (b) 40 Teilen Glycerinmonostearat (Cutina® GMS-V) und (c) 8 Teilen Ceteareth-20 (Eumulgin® B 2) mit einer Geschwindigkeit von 1,8 kg/h/Loch vertropft. Ausgehend von einer Schmelztemperatur von 65 °C stellte sich eine Produkttemperatur von 39 °C ein. Das Abkühlen erfolgte mit 10 °C kalter Luft auf einer Fallhöhe von 11 m. Es wurde ein staubfreies rieselfähiges Granulat erhalten; dieses bestand aus gleichmäßigen Perlen, die zu > 70 % eine Größe von 1 mm aufwiesen. Die bestimmten Auflösegeschwindigkeiten betrugen für die erfindungsgemäßen Perlen 8 min und für die konventionelle geschuppte Ware 15 min.

**Beispiel 11.** In einer Droppo-Line-Anlage der Fa. Rieter Automatik GmbH mit 0,3 mm-Dü-sen wurde eine Mischung (Schmelztemperatur 48 °C) aus (a) 20 Teilen Stearin/Palmi-finsäure (Cutina® FS 45, Cognis Deutschland GmbH), (b) 40 Teilen Cutina® CBS (Mischung konsistenzgebender Wachse) und (c) 10 Teile 4-Methylbenzylidene Camphor (Eusolex® 6300, Merck) mit einer Geschwindigkeit von 1,1 kg/h/Loch vertropft. Ausgehend von einer Schmelztemperatur von 60°C stellte sich eine Produkttemperatur von 31 °C ein. Das Abkühlen erfolgte mit 10°C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,6 mm aufwiesen. Die bestimmten Auflösegeschwindigkeiten betrugen für die erfindungsgemäßen Perlen 18 min und für die konventionelle geschuppte Ware 37 min.

**Beispiel 12.** In einer Droppo-Line-Anlage der Fa. Rieter Automatik GmbH mit 0,3 mm-Düsen wurde eine Mischung (Schmelztemperatur 48 °C) aus (a) 40 Teilen Cetearylalkohol (Lanette® O) und (b) 10 Teilen Benzophenone-3 (NeoHeliopan® BB, Haarmann & Reimer) mit einer Geschwindigkeit von 1,5 kg/h/Loch vertropft. Ausgehend von einer Schmelztemperatur von 60 °C stellte sich eine Produkttemperatur von 34 °C ein. Das Abkühlen erfolgte mit 10°C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,6 mm aufwiesen. Die bestimmten Auflösegeschwindigkeiten betrugen für die erfindungsgemäßen Perlen 9 Minuten und für die konventionelle geschuppte Ware 16 min.

**Beispiel 13**. In einer Droppo-Line-Anlage der Fa. Rieter Automatik GmbH mit 0,3 mm-Düsen wurde eine Mischung (Schmelztemperatur 56 °C) aus (a) 40 Teilen Glycerin-monostearat (Cutina® GMS-V) und (b) Teilen Benzophenone-3 (NeoHeliopan® BB) mit einer Geschwindigkeit von 1,5 kg/h/Loch vertropft. Ausgehend von einer Schmelztemperatur von 70°C stellte sich eine Produkttemperatur von 41 °C ein. Das Abkühlen erfolgte mit 10 °C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,6 mm aufwiesen. Die bestimmten Auflösegeschwindigkeiten betrugen für die erfindungsgemäßen Perlen 10 min und für die konventionell geschuppte Ware 18 min.

**Beispiel 14.** In einer Droppo-Line-Anlage der Fa. Rieter Automatik GmbH mit 0,3 mm-Düsen wurde eine Mischung (Schmelztemperatur 51 °C) aus (a) 45 Teilen Cetearylalkohol (Lanette® O) und (b) 10 Teilen Carotin mit einer Geschwindigkeit von 1,3 kg/h/Loch vertropft. Ausgehend von einer Schmelztemperatur von 67 °C stellte sich eine Produkttemperatur von 38 °C ein. Das Abkühlen erfolgte mit 10 °C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,6 mm aufwiesen. Die bestimmten Auflösegeschwindigkeiten betrugen für die erfindungsgemäßen Perlen 5 min und für die konventionell geschuppte Ware 11 min.

**Beispiel 15.** In einer Droppo-Line-Anlage der Fa. Rieter Automatik GmbH mit 0,3 mm-Düsen wurde eine Mischung (Schmelztemperatur 53 °C) aus (a) 45 Teilen Cetearylalkohol (Lanette® O) und (b) 7 Teilen Natrium-Kokosalkylsulfat (Sulfopon® 1218, Cognis Deutschland GmbH, 65 Gew.-% Aktivsubstanz) mit einer Geschwindigkeit von 1,0 kg/h/Loch vertropft (hierzu wurde die Schmelze über einen Homogenisator (Supraton) dispergiert und auf den Sprühturm geführt) . Ausgehend von einer Schmelzetmperatur von 70 °C stellte sich eine Produkttemperatur von 41 °C ein. Das Abkühlen erfolgte mit 10 °C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,6 mm aufwiesen. Die bestimmten Auflösegeschwindigkeiten betrugen für die erfindungsgemäßen Perlen 7 min und für die konventionelle geschuppte Ware 13 min.

**Beispiel 16.** In einer Droppo-Line-Anlage der Fa. Rieter Automatik GmbH mit 0,3 mm-Düsen wurde eine Mischung (Schmelztemperatur 53 °C) aus (a) 50 Teilen Cetearylalkohol (Lanette® O) und (b) 4 Teilen Kalium-Cetylphosphat (25 Gew.-% Aktivsubstanz) mit einer Geschwindigkeit von 1,1 kg/h/Loch vertropft (hierzu wurde die Schmelze über einen Homogenisator (Supraton) dispergiert und auf den Sprühturm geführt). Ausgehend von einer Schmelztemperatur von 70 °C stellte sich eine Produkttemperatur von 42 °C ein. Das Abkühlen erfolgte mit 10 °C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,6 mm aufwiesen. Die bestimmten Auflösegeschwindigkeiten betrugen für die erfindungsgemäßen Perlen 8 min und für die konventionelle geschuppte Ware 15 min.

**Beispiel 17.** In einer Droppo-Line-Anlage der Fa. Rieter Automatik GmbH mit 0,3 mm-Düsen wurde eine Mischung (Schmelztemperatur 80 °C) aus (a) 70 Teilen einer technischen Kokosalkylpolyglucosid-Mischuing, bestehend aus 80 Gew.-% Glucosid und 20 Gew.-% Fettalkohol) sowie 30 Teilen einer Paste, bestehend aus 50 Gew.-% Kokosalkylpolyglucosid und 50 Gew.-% Wasser (Plantacare® APG 1200, Cognis Deutschland GmbH) mit einer Geschwindigkeit von 0,80 kg/h/Loch vertropft (hierzu wurde die Schmelze über einen Homogenisator (Supraton) dispergiert und auf den Sprühturm geführt). Die Mischung wurde mit einer Temperatur von 95 °C in den Vertropfungs-Sprühkopf eingespeist. Das Abkühlen im Sprühturm mit einer Fallhöhe von 11 m erfolgte mit 10 °C kalter Luft. Es wurde ein staubfreies rieselfähiges Granulat erhalten; dieses bestand aus gleichmäßigen Perlen, die zu > 70 % eine Größe von 0,6 mm aufwiesen. Die Auflösegeschwindigkeit der so erhaltenen Perlen wurde im Vergleich zu einer entsprechenden Ware in geschuppter Form wie folgt getestet: Jeweils 100 g des Perlen- bzw. geschuppten Produktes wurden bei 50°C in 1 Liter Wasser unter Rühren erwärmt, wobei die Perlen bis zu ihrem Lösen nur die Hälfte der Zeit benötigten, welche für das Lösen der geschuppten Ware notwendig war.

**Beispiel 18.** In einer Droppo-Line-Anlage der Fa. Rieter Automatik GmbH mit 0,3 mm-Düsen wurde eine Mischung aus (a) 70 Teilen C_{12/18}-Fettalkohol+20EO und (b) 30 Teilen einer Paste, bestehend aus 50 Gew.-% Kokosalkylpolyglucosid und 50 Gew.-% Wasser (Plantacare® APG 1200) mit einer Geschwindigkeit von 0,90 kg/h/Loch vertropft (hierzu wurde die Schmelze über einen Homogenisator (Supraton) dispergiert und auf den Sprühturm geführt). Die Mischung wurde mit einer Temperatur von 95 °C in den Vertropfungs-Sprühkopf eingespeist. Das Abkühlen im Sprühturm mit einer Fallhöhe von 11 m erfolgte mit 10 °C kalter Luft. Es wurde ein staubfreies rieselfähiges Granulat erhalten; dieses bestand aus gleichmäßigen Perlen, die zu > 70 % eine Größe von 0,6 mm aufwiesen. Ein mit 500 kg/m² zusammengepreßtes Produkt ließ sich nach 2 h Lagerzeit problemlos wieder in Einzelperlen zerteilen. Das Produkt zeigte keine Temperaturerhöhung durch Restwärme. Die Auflösegeschwindigkeit der so erhaltenen Perlen wurde im Vergleich zu einer entsprechenden Ware in geschuppter Form wie folgt getestet: Jeweils 100 g des Perlen- bzw. geschuppten Produktes wurden bei 30 °C in 1 Liter Wasser unter Rühren erwärmt, wobei die Perlen bis zu ihrem Lösen nur die Hälfte der Zeit benötigten, welche für das Lösen der geschuppten Ware notwendig war.

**Beispiel 19.** In einer Droppo-Line-Anlage der Fa. Rieter Automatik GmbH mit 0,3 mm-Düsen wurde eine Mischung (Schmelztemperatur 80 °C) aus (a) 50 Teilen einer technischen Kokosalkylpolyglucosid-Mischuing, bestehend aus 80 Gew.-% Glucosid und 20 Gew.-% Fettalkohol) sowie 50 Teilen C_{12/18}-Fettalkohol+20EO-decylether mit einer Geschwindigkeit von 0,80 kg/h/Loch vertropft (hierzu wurde die Schmelze über einen Homogenisator (Supraton) dispergiert und auf den Sprühturm geführt). Die Mischung wurde mit einer Temperatur von 90 °C in den Vertropfungs-Sprühkopf eingespeist. Das Abkühlen im Sprühturm mit einer Fallhöhe von 11 m erfolgte mit 10 °C kalter Luft. Es wurde ein staubfreies rieselfähiges Granulat erhalten; dieses bestand aus gleichmäßigen Perlen, die zu > 70 % eine Größe von 0,6 mm aufwiesen Die Auflösegeschwindigkeit der so erhaltenen Perlen wurde im Vergleich zu einer entsprechenden Ware in geschuppter Form wie folgt getestet: Jeweils 100 g des Perlen- bzw. geschuppten Produktes wurden bei 50 °C in 1 Liter Wasser unter Rühren erwärmt, wobei die Perlen bis zu ihrem Lösen nur die Hälfte der Zeit benötigten, welche für das Lösen der geschuppten Ware notwendig war.

**Beispiel 20.** In einer Droppo-Line-Anlage der Fa. Rieter Automatik GmbH mit 0,3 mm-Düsen wurde Stearinsäure+40EOmethylester (Schmelztemperatur 51 °C) mit einer Geschwindigkeit von 0,7 kg/h/Loch vertropft. Ausgehend von einer Schmelztemperatur von 65 °C stellte sich eine Produkttemperatur von 24 °C ein. Das Abkühlen erfolgte mit 5 °C kalter Luft auf einer Fallhöhe von 11 m. Es wurde ein staubfreies rieselfähiges Granulat erhalten; dieses bestand aus gleichmäßigen Perlen, die zu > 70 % eine Größe von 0,6 mm aufwiesen. Die Auflösegeschwindigkeit der so erhaltenen Perlen wurde im Vergleich zu einer entsprechenden Ware in geschuppter Form wie folgt getestet: Jeweils 100 g des Perlen- bzw. geschuppten Produktes wurden bei 50 °C in 1 Liter Wasser unter Rühren erwärmt, wobei die Perlen bis zu ihrem Lösen nur die Hälfte der Zeit benötigten, welche für das Lösen der geschuppten Ware notwendig war.

**Beispiel 21.** In einer Droppo-Line-Anlage der Fa. Rieter. Automatik GmbH mit 0,5 mm-Düsen wurde eine Mischung (Schmelztemperatur 45 °C) aus (a) 50 Teilen Kokosfettsäure+10EO-methylester und (b) 50 Teilen Polyethylenglycol (Molgewicht 4.000) mit einer Geschwindigkeit von 1,7 kg/h/Loch vertropft. Ausgehend von einer Schmelztemperatur von 75 °C stellte sich eine Produkttemperatur von 34 °C ein. Das Abkühlen erfolgte mit 15 °C kalter Luft auf einer Fallhöhe von 11 m. Die erhaltenen Tropfen wurden anschließend in Kieselsäure abgepudert. Es wurde ein staubfreies rieselfähiges Granulat erhalten; dieses bestand aus gleichmäßigen Perlen, die zu > 70 % eine Größe von 1 mm aufwiesen. Die Auflösegeschwindigkeit der so erhaltenen Perlen wurde im Vergleich zu einer entsprechenden Ware in geschuppter Form wie folgt getestet: Jeweils 100 g des Perlen- bzw. geschuppten Produktes wurden bei 50 °C in 1 Liter Wasser unter Rühren erwärmt, wobei die Perlen bis zu ihrem Lösen nur die Hälfte der Zeit benötigten, welche für das Lösen der geschuppten Ware notwendig war.

## Patentansprüche

1. Feststoffgranulate mit einer monodispersen Korngrößenverteilung, **dadurch** erhältlich, dass man eine bei Raumtemperatur feste, überwiegend wasserfreie Zubereitung aus mindestens einem kosmetischen Einsatzstoff ausgewählt aus der Gruppe nichtionischer Tenside, die gebildet wird von Alkyl- und/oder Alkenyloligoglykosiden, Fettsäure-N-alkylpoly-hydroxyalkylamiden, Fettalkoholpolyglycolethern, Mischethern, alkoxylierten Fettsäurealkylestern und Polyolpoly-12-hydroxystearaten, verflüssigt, einen Strom der Schmelze mit Hilfe einer Gießerplatte durch Vibration vertropft und den Tropfen ein Kühlmittel entgegenleitet, welches diese zum Erstarren bringt.

2. Verfahren zur Herstellung von Feststoffgranulaten mit einer monodispersen Korngrößenverteilung, bei dem man eine bei Raumtemperatur feste, überwiegend wasserfreie Zubereitung aus mindestens einem kosmetischen Einsatzstoff verflüssigt, einen Strom der Schmelze mit Hilfe einer Gießerplatte durch Vibration vertropft und den Tropfen ein Kühlmittel entgegenleitet, welches diese zum Erstarren bringt.

3. Verfahren nach Anspruch 2, **dadurch gekennzeichnet, dass** man kosmetischen Einsatzstoffe einsetzt, die ausgewählt sind aus der Gruppe, die gebildet wird von Emulgatoren, Wachskörpem und Konsistenzgebem.

4. Verfahren nach Anspruch 2 bis 3, **dadurch gekennzeichnet, dass** man als Emulgatoren nichtionische Tenside einsetzt, die ausgewählt sind aus der Gruppe, die gebildet wird von Alkyl- und/oder Alkenyloligoglykosiden, Fettsäure-N-alkylpoly-hydroxyalkylamiden, Fettalkoholpolyglycolethern, Mischethern, alkoxylierten Fettsäure-alkylestern und Polyolpoly-12-hydroxystearaten.

5. Verfahren nach mindestens einem der Ansprüche 2 bis 4, **dadurch gekennzeichnet, dass** man Wachskörper einsetzt, die ausgewählt sind aus der Gruppe, die gebildet wird von natürlichen Wachsen, synthetischen Wachsen, Lecithinen, Phospholipiden und Perlglanzwachsen.

6. Verfahren nach mindestens einem der Ansprüche 2 bis 5, **dadurch gekennzeichnet, dass** man als Konsistenzgeber Fettalkohole und/oder Partialglyceride einsetzt.

7. Verfahren nach mindestens einem der Ansprüche 2 bis 6, **dadurch gekennzeichnet, dass** man Zubereitungen einsetzt, welche weniger als 25 Gew.-% Wasser enthalten.

8. Verfahren nach mindestens einem der Ansprüche 2 bis 7, **dadurch gekennzeichnet, daß** man die kosmetischen Einsatzstoffe zusammen mit weiteren Zusatzstoffen einsetzt, welche ausgewählt sind aus der Gruppe, die gebildet wird von UV-Lichtschutz-filtern, Antioxidantien und biogenen Wirkstoffen.

9. Verfahren nach mindestens einem der Ansprüche 2 bis 8, **dadurch gekennzeichnet, daß** die Zubereitungen mit einer Frequenz von 100 bis 1000 Hz schwingen.

10. Verwendung von Feststoffgranulaten nach Anspruch 1 zur Herstellung von oberflächenaktiven Zubereitungen.

## Claims

1. A granular solid with a monodisperse particle size distribution, obtainable by liquefying a predominantly anhydrous preparation which is solid at room temperature and comprises at least one cosmetic feed material selected from the group of nonionic surfactants chosen from the group formed by alkyl and/or alkenyl oligoglycosides, fatty acid N-alkylpolyhydroxyalkylamides, fatty alcohol polyglycol ethers, mixed ethers, alkoxylated fatty acid alkyl esters and polyol poly-12-hydroxystearates, making a stream of the melt into droplets using a casting plate by vibration, and passing a cooling medium countercurrently to the droplets, which causes them to solidify.

2. A process for the preparation of granular solids with a monodisperse particle size distribution in which a predominantly anhydrous preparation which is solid at room temperature and which comprises at least one cosmetic feed material is liquefied, a stream of the melt is made into droplets using a casting plate by vibration and a cooling medium is passed countercurrently to the droplets, which causes them to solidify.

3. The process as claimed in claim 2, **characterized in that** cosmetic feed materials are used which are chosen from the group formed by emulsifiers, wax bodies and bodying agents.

4. The process as claimed in claim 2 and/or 3, **characterized in that** the emulsifiers used are nonionic surfactants chosen from the group formed by alkyl and/or alkenyl oligoglycosides, fatty acid N-alkylpolyhydroxyalkylamides, fatty alcohol polyglycol ethers, mixed ethers, alkoxylated fatty acid alkyl esters and polyol poly-12-hydroxystearates.

5. The process as claimed in at least one of claims 2 to 4, **characterized in that** wax bodies are used which are chosen from the group formed by natural waxes, synthetic waxes, lecithins, phospholipids and pearlescent waxes.

6. The process as claimed in at least one of claims 2 to 5, **characterized in that** the bodying agents used are fatty alcohols and/or partial glycerides.

7. The process as claimed in at least one of claims 2 to 6, **characterized in that** preparations are used which comprise less than 25% by weight of water.

8. The process as claimed in at least one of claims 2 to 7, **characterized in that** the cosmetic feed materials are used together with further additives which are chosen from the group formed by UV light protection filters, antioxidants and biogenic active ingredients.

9. The process as claimed in at least one of claims 2 to 8, **characterized in that** the preparations oscillate at a frequency of from 100 to 1 000 Hz.

10. The use of granular solids as claimed in claim 1 for the preparation of surface-active preparations.

## Revendications

1. Granulats solides présentant une répartition granulométrique monodispersée, pouvant être obtenus en ce qu'on liquéfie une composition solide à température ambiante, principalement anhydre, constituée par au moins une substance cosmétique choisie dans le groupe des agents tensioactifs non ioniques, qui est formé par les alkyloligoglycosides et/ou les alcényloligoglycosides, les N-alkylpolyhydroxyalkylamides d'acides gras, les polyglycoléthers d'alcools gras, les éthers mixtes, les esters alkyliques d'acides gras alcoxylés et les poly-12-hydroxystéarates de polyol, on transforme un flux de la masse fondue en gouttes par vibration à l'aide d'une plaque de coulée et on guide les gouttes à contrecourant d'un agent de refroidissement, qui les solidifie.

2. Procédé pour la préparation de granulats solides présentant une répartition granulométrique monodispersée, dans lequel on liquéfie une composition solide à température ambiante, principalement anhydre, constituée par au moins une substance cosmétique, on transforme un flux de la masse fondue en gouttes par vibration au moyen d'une plaque de coulée et on guide les gouttes à contrecourant d'un agent de refroidissement, qui les solidifie.

3. Procédé selon la revendication 2, **caractérisé en ce qu'**on utilise des substances cosmétiques qui sont choisies dans le groupe formé par les émulsifiants, les corps cireux et les adjuvants conférant une consistance.

4. Procédé selon la revendication 2 à 3,
**caractérisé en ce qu'**on utilise, comme émulsifiants, des agents tensioactifs non ioniques, qui sont choisis dans le groupe formé par les alkyloligoglycosides et/ou les alcényloligoglycosides, les N-alkylpolyhydroxyalkylamides d'acides gras, les polyglycoléthers d'alcools gras, les éthers mixtes, les esters alkyliques d'acides gras alcoxylés et les poly-12-hydroxystéarates de polyol.

5. Procédé selon au moins l'une quelconque des revendications 2 à 4, **caractérisé en ce qu'**on utilise des corps cireux qui sont choisis dans le groupe formé par les cires naturelles, les cires synthétiques, les lécithines, les phospholipides et les cires nacrées.

6. Procédé selon au moins l'une quelconque des revendications 2 à 5, **caractérisé en ce qu'**on utilise comme adjuvants conférant une consistance des alcools gras et/ou des glycérides partiels.

7. Procédé selon au moins l'une quelconque des revendications 2 à 6, **caractérisé en ce qu'**on utilise des compositions qui contiennent moins de 25% en poids d'eau.

8. Procédé selon au moins l'une quelconque des revendications 2 à 7, **caractérisé en ce qu'**on utilise les substances cosmétiques ensemble avec d'autres additifs qui sont choisis dans le groupe formé par les filtres de protection contre la lumière UV, les antioxydants et les substances actives biogènes.

9. Procédé selon au moins l'une quelconque des revendications 2 à 8, **caractérisé en ce que** les compositions oscillent à une fréquence de 100 à 1000 Hz.

10. Utilisation de granulats solides selon la revendication 1 pour la préparation de compositions tensioactives.
